# EUROPEAN PATENT SPECIFICATION

(11) **EP 3 110 781 B1**
(45) Date of publication and mention of the grant of the patent: **27.06.2018**
(21) Application number: 15714282.9
(22) Date of filing: 26.02.2015
(51) Int. Cl.: C07C 29/128, C07C 29/80, C07C 29/86, C07C 31/02

(54) **MICROWAVE-ASSISTED TREATMENT FOR THE PRODUCTION OF POLYCOSANOL-ENRICHED MIXTURES FROM WAX MATRICES**
MIKROWELLENUNTERSTÜTZTE BEHANDLUNG ZUR HERSTELLUNG VON POLYCOSANOL-ANGEREICHERTEN MISCHUNGEN AUS WACHSMATRIZEN
TRAITEMENT ASSISTÉ PAR MICRO-ONDES POUR LA PRODUCTION DE MÉLANGES ENRICHIS EN POLICOSANOL PROVENANT DE MATRICES DE CIRE

(30) Priority: 27.02.2014 IT MI20140304
(43) Date of publication of application: 04.01.2017
(73) Proprietor: Tydock Pharma S.r.l., 41126 Modena (IT)
(72) Inventor: PELLATI, Federica, I-41125 Modena (IT); MASCOLO, Danilo, I-40125 Bologna (IT); VENTURELLI, Alberto, I-41053 Maranello (Modena) (IT); BARBATO, Amedeo, I-80055 Portici (Napoli) (IT); RIGHI, Davide, I-41123 Modena (IT); CASTALDI, Paco, I-83020 Sirignano (Avellino) (IT)
(74) Representative: Biggi, Cristina
(86) International application number: PCT/IB2015/051432
(87) International publication number: WO 2015/128829

(56) References cited:
- IN-A1- 188 438
- JEYASHOKE N ET AL: "Microwave induced rapid transmethylation of fatty acids for analysis of food oil", JOURNAL OF CHROMATOGRAPHY, ELSEVIER SCIENCE PUBLISHERS B.V, NL, vol. 818, no. 1, 28 August 1998 (1998-08-28), pages 133-137, XP004145874, ISSN: 0021-9673, DOI: 10.1016/S0021-9673(98)00545-7

## Description

### TECHNICAL FIELD

The present invention relates to an economical process for the production of large quantities of aliphatic alcohols with a chain length of 20 to 36 carbon atoms, in particular for the production of policosanols.

### PRIOR ART

Policosanols derive from natural waxes of both animal and vegetable origin. At present there is considerable interest in seeking out new methods of extracting these compounds. This is due to the fact that there are numerous benefits for human health linked to the use of policosanols which have been reported in the literature; the most interesting research findings in this area include:
1. Policosanols have an activity of reducing cholesterol in experimental animal models and in human subjects affected by type II hypercholesterolemia. Their action in the pharmacological/clinical realm has been studied along with their therapeutic importance as hypocholesterolemizing agents; the results have been described by international research groups (Gouni-Berthold I et al., Am Heart J. 2002 143:356-65).
2. Policosanols are considered a promising resource in the prevention and treatment of cardiovascular diseases (CVD) (Varady KA, Wang Y, Jones PJ. Nutr Rev. 2003, 61 (II):376-83).
3. Policosanols are more effective than plant sterols and stanols and are moreover more active than the same in reducing LDL levels, as well as improving the lipid profile (Chen JT, et al., Pharmacotherapy 2005, 25(2):171-83).
4. Policosanols have activity in reducing the formation of atherosclerotic plaques and in the stability thereof; this could explain their protective effect against the development of atherosclerosis (Noa M, Mas R, Arch Med Res. 2005, 36(5):441-7).
5. Policosanols or their metabolites decrease the activity of HMG-CoA reductase whilst activating AMP-kinase (Singh DK, Li L, Porter TD, J Pharmacol Exp Ther. 2006, 318(3):1020-6).
6. Policosanols have protective activity in patients susceptible to heart attack (Ortega LL, et al., J Med Food 2006, 9(3):378-85).
7. Policosanols demonstrate an anticoagulant effect and prevent atherosclerotic lesions induced by lipofundin (Noa M, Mas R, Lariot C, J Med Food 2007, 10(3):452-9).

By virtue of the listed properties, policosanols are very promising molecules and represent an added value in pharmaceutical and nutraceutical products and in the food and cosmetics sectors, with a demand that is increasing day by day.

There are numerous known processes that provide policosanols on an industrial level, where they are isolated from a large variety of vegetable materials.
1. Horn A. and Martic J.S. describe a method for obtaining aliphatic alcohols from sugar cane cuticular wax by homogeneous saponification with an alcoholic solution of potassium hydroxide, followed by esterification or acetylation of the residual unsaponified material and, finally, purification by molecular distillation (Horn A., Martic J.S., 1957, Journal of the Science of Food and Agriculture, 10, 571).
2. Inada S. et al. propose a process for extracting 20 aliphatic alcohols from the waxy material of sugar cane using carbon dioxide (CO₂) and ethylene under sub- and supercritical conditions to obtain a mixture with 7-10% octacosanol (C28) and 0.4-1% triacontanol (C30) as the principal components (JP 60-119514,1986).
3. Bertholet describe a method for preparing policosanols by saponification from rice bran wax, jojoba oil and carnauba wax. The overall yield of the reaction is approximately 50% (Bertholet, 1991, U.S. Pat. No. 5,159,124).
4. Laguna et al. obtain a mixture of aliphatic alcohols having chains of 24 to 34 carbon atoms by saponification of sugar cane wax. The yield is approximately 30% (Laguna et al., 1996, U.S. Pat. No. 5,856,316).
5. Granja et al. describe a process for obtaining policosanols with 22 to 38 carbon atoms; the process entails saponifying and extracting alcohols using organic solvents (Granja et al., 1997, U.S. Pat. No. 5,663,156).
6. Steel et al. propose extracting n-hexacosanol (C26) or an n-hexacosanol-rich mixture containing C20 to C32 alcohols. Lanolin or sheep's wool are subjected to hydrolysis and subsequently extracted or concentrated (Steel et al., 1999, WO1999048853A1).
7. Perez has developed a method for isolating a mixture of primary aliphatic alcohols from non-alcoholic compounds contained in beeswax by subjecting unsaponified beeswax to extraction and purification with organic solvents. The yield of alcohols obtained is around 35% (Perez et al., 2001, U.S. Pat. No. 6,225,354).
8. Gamble has developed a method for obtaining policosanols by extraction with an organic solvent (Gamble, 2003, U.S. Pat. No. 6,596,776).
9. Jia et al. propose a method for extracting a mixture of policosanols having from 24 to 30 carbon atoms from the wax of the insect *Ericerus pela* by hydrolyzing the melted wax obtained with a base and subsequently neutralizing the hydrolysate (Jia, Qi, 2004, U. S. Pat. No. 5 6,683,116).
10. Empie discloses a method for extracting policosanols from seeds, vegetables or waste materials, including, for example, waste products from the processing of soybean, by using solid-liquid extraction (Empie, Mark W., 2007, U.S. Pat. No. 7214394).
11. Majeed uses supercritical CO₂ extraction and immobilized lipase to saponify and obtain, in a single process, pure alcohols with 24 to 36 carbon atoms (Majeed, 2007, U.S. Pat. No. 7,217,546).

Most of the known processes are based on saponification or hydrolysis. These processes are responsible for the formation of large quantities of insoluble impurities (mostly polymers and organic salts), which inevitably influence the total yield. The other methods, such as extraction with supercritical fluids, possibly with enzymes, are not suited to processes conducted on a medium/large scale due to the high operating costs. Furthermore, direct extraction with organic solvents cannot be applied because of the considerable time required. Finally, many of these processes can have a very low yield. IN 188 438 A1 discloses a process for preparing triacontanol wherein sugarcane wax is subjected to a transesterification.

The invention aims to solve the problems found with prior art processes by providing a method for extracting policosanols from wax or waxy materials characterized by better extraction yields compared to prior art processes and a better industrial scalability.

### SUMMARY OF THE INVENTION

The object of the invention is to provide a method for obtaining a mixture of policosanols from waxy material, in particular wax, originating from animal or vegetable sources. Preferably, the waxy material originates from insects, in particular bees. The present invention relates to a process for obtaining a mixture of policosanols as defined in claim 1. The entire process benefits from two steps developed using microwaves, which have never been applied in this field. The solvents used in the process can be largely recovered by distillation.

The process of the invention moreover enables an extremely pure mixture of policosanols to be obtained with a high extraction yield. It is in fact the aim of this invention to provide a mixture of purified policosanols with a purity comprised from 90% to 100%. Preferably, the mixture of policosanols comprises: 1-eicosanol (C20), in an amount of 0-5% by weight, 1-docosanol (C22), 0-5% by weight, 1-tetracosanol (C24), 0-5% by weight, 1-hexacosanol (C26), 0-5% by weight, 1-octacosanol (C28), 5-10% by weight, 1-triacontanol (C30), 28-35% by weight, 1-dotriacontanol (C32), 23-28% by weight, and 1-tetratriacontanol (C34), 0-5% by weight.

The mixture of policosanols obtained with the method of the invention can be utilized as such as an ingredient for pharmaceutical, nutraceutical, food or cosmetic uses, etc., or else it can be used to obtain the individual policosanol of interest through suitable purification processes known in the art, to be employed as an ingredient in different pharmaceutical, nutraceutical, food or cosmetic products, etc..

### BRIEF DESCRIPTION OF THE FIGURES

The invention will be illustrated below also with reference to the experimental part, in which the figures appended hereto will be commented on:
- Figure 1 shows a typical chromatogram of yellow beeswax;
- Figure 2 shows a chromatogram of the transesterified mixture;
- Figure 3 shows a chromatogram of the mixture after hydrolysis;
- Figure 4 shows a chromatogram of the final product after the two microwave-assisted processes and the treatment with an organic solvent.

### DETAILED DESCRIPTION OF THE INVENTION

The present invention is based on a process for obtaining a mixture of long-chain aliphatic alcohols, in particular a mixture of policosanols, from waxy material, in particular wax, originating from animal or vegetable sources, characterized by a high policosanol extraction yield and purity in policosanols of the extract obtained. The starting waxy materials naturally contain a mixture of policosanols having 20 to 36 carbon atoms; most of these are present in a form in which they are esterified with fatty acids in the starting material. Through this invention, comprising at least two microwave-assisted transesterification and hydrolysis steps, and other classic purification techniques, it is possible to obtain free policosanols more quickly and in a larger quantity.

The process comprises the following steps: (1) microwave-assisted transesterification of the waxy material or wax in an alcoholic environment with a basic pH obtained using a base chosen in the group consisting of potassium hydroxide, sodium hydroxide, ammonium hydroxide, calcium hydroxide and calcium carbonate; (2) washing of the transesterified material with water to remove the water-soluble salified fatty acids; (3) microwave-assisted hydrolysis in an alcoholic environment with a basic pH to purify the policosanols of the unreacted fatty acids and of esters; (4) purification with non-polar organic solvents. This final step is responsible for the increase in the purity of the policosanols, thanks to the removal of undesirable components such as natural paraffins, a group of long-chain alkanes originating from the starting material.

In a preferred embodiment, the transesterification step (1) is preferably preceded by a preparation of the waxy material (by division into scales) such as to increase the contact surface thereof.

In another preferred embodiment, after transesterification the transesterified waxy material preferably undergoes filtration and possible recovery of the alcoholic phase by distillation or equivalent methods.

In the microwave-assisted steps, the pressures and temperatures are controlled. The starting material from which high-purity policosanols are obtained, according to the process of the invention, are waxy materials of natural origin, in particular waxes of animal derivation (for example, beeswax) or vegetable derivation (for example, sugar cane, wheat germ, rice bran etc.).

Policosanols are a mixture of long-chain aliphatic alcohols whose composition depends on the material they are extracted from and the type of process. The main alcohols obtainable with the process of the invention are aliphatic alcohols comprising 20 to 36 carbon atoms, for example tetracosanol (C24), hexacosanol (C26), octacosanol (C28), triacontanol (C30) and dotriacontanol (C32).

The first step entails subjecting the starting waxy material to a homogeneous transesterification. For this purpose the waxy material, preferably reduced into scales, is placed in a microwave reactor in an alcoholic environment with a basic pH; during the process a medium stirring speed is applied. The alcohol used is preferably ethanol (96% or 99%); other examples of utilizable solvents include, but are not exclusively limited to, methanol, propanol, butanol, etc.. The base used is preferably potassium hydroxide; other examples of utilizable bases include sodium hydroxide, ammonium hydroxide, calcium hydroxide and calcium carbonate. The ratio of the waxy material (g) relative to the alcohol (mL) is in the range of 1:0.05 to 1:0.5, preferably 1:0.15.

In particular, the waxy material and the described mixture are placed in the container for microwaves, so as then to be subjected to the microwave treatment. One example of experimental parameters utilizable for the transesterification is the following:
1. Irradiation power (Max): 200 Watts
2. Applied pressure (Max): 15.9 bar (230 psi)
3. Applied temperature: 120 °C
4. Stirring speed: medium
5. Reaction time: 1 min ramp time followed by 1 min hold time.
6. Pressure: during the ramp time the pressure reaches a value of 15.2 bar (220 psi) and a power of 165 Watts; this is followed by a constant pressure of 6.89 bar (100 psi) during the hold time and a power of 40 Watts.

After the transesterified product has been cooled to room temperature, the solid is preferably vacuum filtered; the insoluble part of the mixture on the filter is washed with water and subjected to drying in an oven. The ratio between the volume of water (mL) and the sample weight (g) can vary from 1:50 to 1:5000, preferably it is 1:500. The alcoholic phase can be recovered by distillation.

The transesterified mixture obtained after the first step then undergoes microwave-assisted hydrolysis in an alcoholic environment with a basic pH.
The conditions and parameters of the microwave system are, for example, the same as described for the first reaction, with the exception of the hold time, which, for example, is set at 20 min.
The preferred ratio between the transesterified product (g) and alcohol (mL), preferably ethanol, is in the range of 1:5 to 1:50, more preferably 1:15. In this step a basic solution of sodium hydroxide (or the other previously mentioned bases) is preferably added. The ratio of transesterified product (g) to (mL) of basic solution can vary from 1:5 to 1:50, more preferably it is 1:15.
The product obtained from this second step is preferably vacuum filtered; the solid product is then washed with water to remove the undesirable soluble components. The volume of water used for washing is the same as in the first step. The alcoholic solvent can be recovered by distillation.

The final purification step (4) can be performed by dissolving the hydrolyzed mixture under heat in non-polar organic solvents, preferably heptane; other examples of utilizable solvents include, but are not exclusively limited to, pentane, hexane, octane, cyclohexane, toluene, diethyl ether, petroleum ether, etc.. A hydrolyzed mixture/solvent ratio of 0.5 g/100 mL to 5 g/100 mL, preferably 1 g/100 mL, is used. The solution is allowed to cool to room temperature; it is then vacuum filtered and dried. The organic solvent can be recovered by distillation.

The process of the invention makes it possible to obtain purified policosanols with a purity comprised from 90% to 100%.
The last step makes it possible to obtain, for example, a GC-MS composition (obtained after derivatization with MSTFA+1 %TMS) as follows: 0-5% 1-eicosanol (C20), 0-5% 1-docosanol (C22), 0-5% 1-tetracosanol (C24), 0-5% 1-hexacosanol (C26), 5-10% 1-octacosanol (C28), 28-35% 1-triacontanol (C30), 23-28% 1-dotriacontanol (C32) and 0-5% 1-tetratriacontanol (C34).

### Example

Below is an example of the process developed in the two steps (A+B) on a 200 mg microwave-processed beeswax sample subsequently subjected to final purification (C) with solvent.

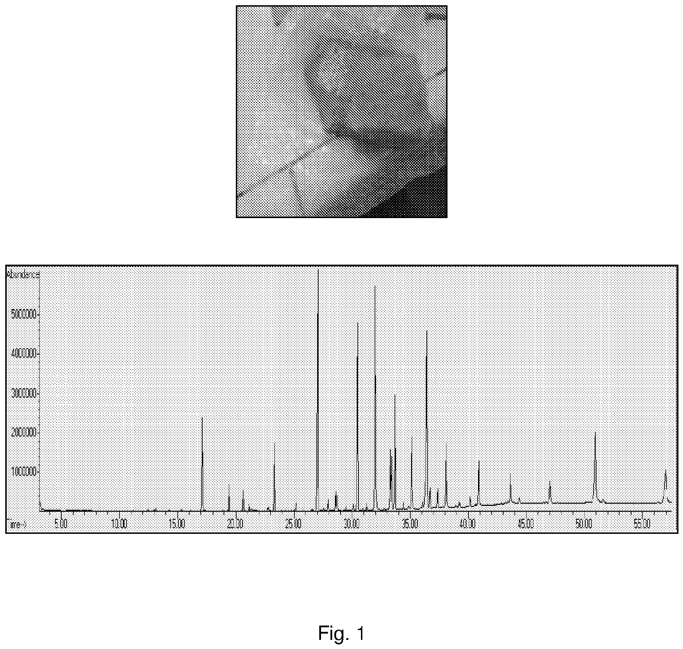

Figure 1 shows a typical chromatogram of yellow beeswax.

All of the GC-MS chromatograms were obtained following derivatization of the sample with MSTFA+1%TMS.

### MICROWAVE-ASSISTED TRANSESTERIFICATION

Two hundred milligrams of yellow beeswax is placed in a closed vessel and subjected to microwave-assisted transesterification (the experimental conditions are described in the section headed "detailed description of the invention") with 3 mL of 96% ethanol and 30 mg of potassium hydroxide. After cooling to room temperature, the mixture obtained is vacuum filtered; the solid recovered on the filter is washed with 100 mL of water and then dried in an oven for 1 hour at 150 °C. The alcoholic solvent is recovered by distillation.

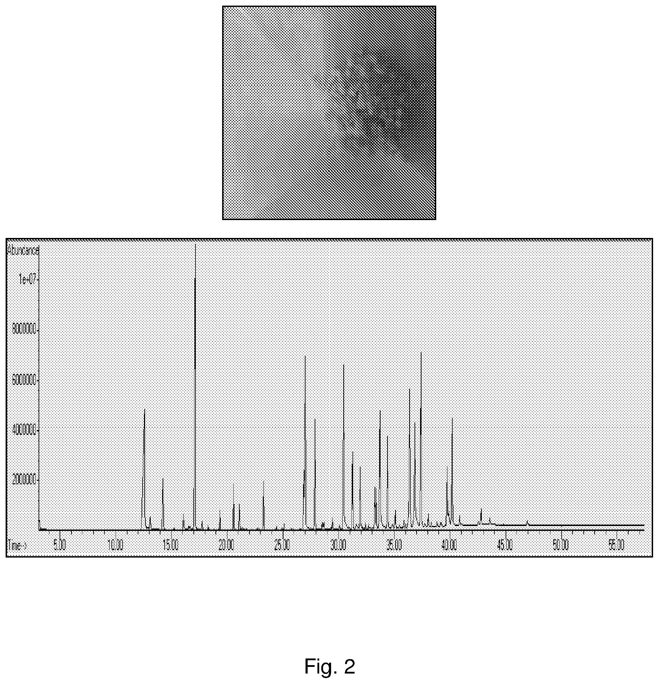

Figure 2 shows a chromatogram of the transesterified mixture.

### MICROWAVE-ASSISTED HYDROLYSIS

One hundred milligrams of transesterified mixture is placed in a closed vessel with 1.5 mL of 96% ethanol and 1.5 mL of 2 M sodium hydroxide solution. The conditions of the microwave system and the parameters applied are the same as previously described with the exception of the hold time, which is set at 20 min. After the reaction, the solid is filtered as previously described and washed with 100 mL of water. The alcoholic solvent is recovered by distillation.

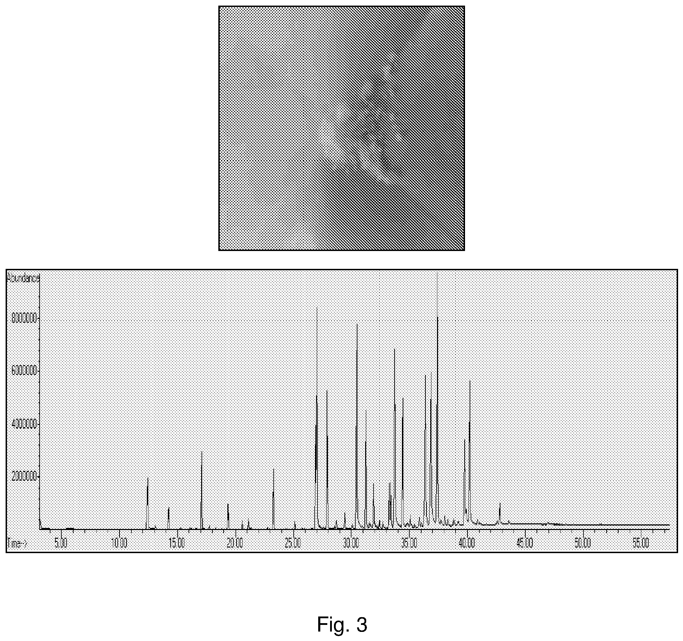

Figure 3 shows a chromatogram of the mixture after hydrolysis.

### PURIFICATION WITH ORGANIC SOLVENT

Finally, the hydrolyzed mixture is dissolved under heat in 10 mL of heptane and then allowed to cool to room temperature. Subsequently, the purified solid product is vacuum filtered using a Buchner funnel with filter paper. Finally, the heptane is recovered by distillation.

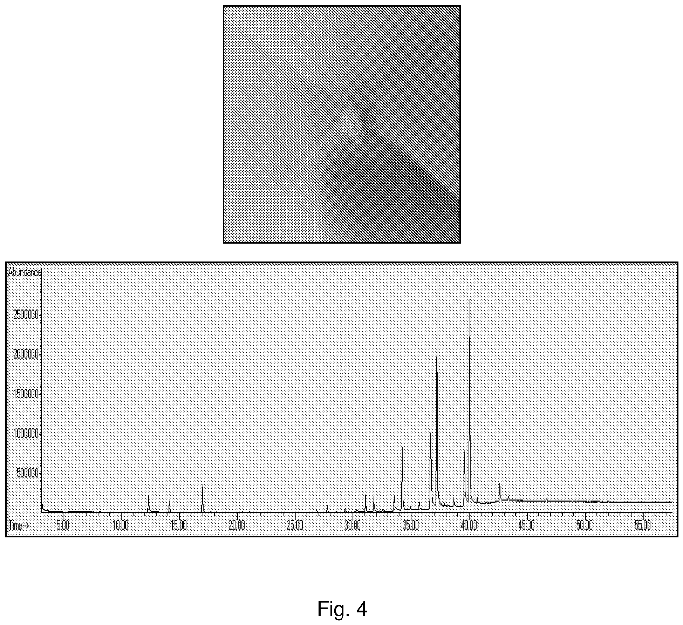

Figure 4 shows the final product after the two microwave-assisted processes and the treatment with organic solvent.

## Claims

1. A process for obtaining a mixture of policosanols from waxy material, comprising the following steps: (1) microwave-assisted transesterification of the waxy material in an alcoholic environment with a basic pH obtained using a base chosen in the group consisting of potassium hydroxide, sodium hydroxide, ammonium hydroxide, calcium hydroxide and calcium carbonate; (2) washing of the transesterified material with water to remove the water-soluble salified fatty acids; (3) microwave-assisted hydrolysis in an alcoholic environment with a basic pH to purify the transesterified material of the unreacted fatty acids and of esters; (4) purification with non-polar organic solvents.

2. The process according claim 1, wherein said waxy material originates from animal or vegetable sources, preferably from beeswax, sugar cane, wheat germ or rice bran.

3. The process according claim 1, wherein after the transesterification step, the transesterified material undergoes filtration and possible recovery of the alcoholic phase.

4. The process according claim 1, wherein the ratio of waxy material (g) to alcohol (mL) is in the range of 1:0.05 to 1:0.5, preferably 1:0.15.

5. The process according to claim 1, wherein the ratio of transesterified product (g) to alcohol (mL) is from 1:5 to 1:50, preferably 1:15.

6. The process according to any one of claims 1 to 5, wherein the hydrolyzed material is vacuum filtered and washed with water to remove the undesirable soluble components.

7. The process according to any one of claims 1 to 6, wherein in the purification step (4), the hydrolyzed mixture is dissolved under heat in non-polar organic solvents, preferably heptane, pentane, hexane, octane, cyclohexane or toluene.

8. The process according to any one of claims 1 to 7, wherein the organic solvents used in the processing steps are recovered by distillation.

## Patentansprüche

1. Verfahren zur Herstellung einer Mischung von Policosanolen aus wachsartigem Material, umfassend die folgenden Schritte: (1) mikrowellenunterstützte Umesterung des wachsartigen Materials in einer alkoholischen Umgebung mit einem basischen pH-Wert, erhalten unter Verwendung einer Base, ausgewählt aus der Gruppe bestehend aus Kaliumhydroxid, Natriumhydroxid, Ammoniumhydroxid, Calciumhydroxid und Calciumcarbonat; (2) Waschen des umgeesterten Materials mit Wasser zur Entfernung der wasserlöslichen, salzhaltigen Fettsäuren; (3) mikrowellenunterstützte Hydrolyse in einer alkoholischen Umgebung mit einem basischen pH-Wert zur Reinigung des umgeesterten Materials der nicht umgesetzten Fettsäuren und der Ester; (4) Reinigung mit unpolaren organischen Lösungsmitteln.

2. Verfahren nach Anspruch 1, wobei das wachsartige Material aus tierischen oder pflanzlichen Quellen stammt, vorzugsweise aus Bienenwachs, Zuckerrohr, Weizenkeimen oder Reiskleie.

3. Verfahren nach Anspruch 1, wobei das umgeesterte Material nach dem Umesterungsschritt einer Filtration und einer möglichen Rückgewinnung der alkoholischen Phase unterzogen wird.

4. Verfahren nach Anspruch 1, wobei das Verhältnis von wachsartigem Material (g) zu Alkohol (mL) im Bereich von 1:0,05 bis 1:0,5, vorzugsweise 1:0,15, liegt.

5. Verfahren nach Anspruch 1, wobei das Verhältnis vom umgeesterten Produkt (g) zu Alkohol (mL) 1:5 bis 1:50, vorzugsweise 1:15, liegt.

6. Verfahren nach einem der Ansprüche 1 bis 5, wobei das hydrolysierte Material unter Vakuum gefiltert und mit Wasser gewaschen wird, um die unerwünschten löslichen Bestandteile zu entfernen.

7. Verfahren nach einem der Ansprüche 1 bis 6, wobei im Reinigungsschritt (4) die hydrolysierte Mischung unter Wärme in unpolaren, organischen Lösungsmitteln, vorzugsweise Heptan, Pentan, Hexan, Oktan, Cyclohexan oder Toluol, gelöst wird.

8. Verfahren nach einem der Ansprüche 1 bis 7, wobei die in den Verfahrensschritten verwendeten organischen Lösungsmittel durch Destillation gewonnen werden.

## Revendications

1. Procédé servant à obtenir un mélange de policosanols provenant de matière cireuse, comprenant les étapes suivantes : (1) la transestérification assistée par micro-ondes de la matière cireuse dans un environnement alcoolique avec un pH basique obtenu en utilisant une base choisie dans le groupe consistant en de l'hydroxyde de potassium, de l'hydroxyde de sodium, de l'hydroxyde d'ammonium, de l'hydroxyde de calcium et du carbonate de calcium ; (2) le lavage de la matière transestérifiée avec de l'eau pour retirer les acides gras salifiés solubles dans l'eau ; (3) une hydrolyse assistée par micro-ondes dans un environnement alcoolique avec un pH basique pour purifier la matière transestérifiée des acides gras inaltérés et des esters ; (4) une purification avec des solvants organiques apolaires.

2. Procédé selon la revendication 1, dans lequel ladite matière cireuse provient de sources animales ou végétales, de préférence de cire d'abeilles, de canne à sucre, de germe de blé ou de son de riz.

3. Procédé selon la revendication 1, dans lequel après l'étape de transestérification, la matière transestérifiée subit une filtration et un rétablissement possible de la phase alcoolique.

4. Procédé selon la revendication 1, dans lequel le rapport de matière cireuse (g) à l'alcool (mL) est compris entre 1:0,05 et 1:0,5, de préférence 1:0,15.

5. Procédé selon la revendication 1, dans lequel le rapport de produit transestérifié (g) à l'alcool (mL) est compris entre 1:5 et 1:50, de préférence 1:15.

6. Procédé selon l'une quelconque des revendications de 1 à 5, dans lequel la matière hydrolysée est filtrée sous vide et lavée avec de l'eau pour retirer les composants solubles indésirables.

7. Procédé selon l'une quelconque des revendications de 1 à 6, dans lequel dans l'étape de purification (4), le mélange hydrolysé est dissous sous l'effet de la chaleur dans des solvants organiques apolaires, de préférence de l'heptane, du pentane, de l'hexane, de l'octane, du cyclohexane ou du toluène.

8. Procédé selon l'une quelconque des revendications de 1 à 7, dans lequel les solvants organiques utilisés dans les étapes de traitement sont récupérés par distillation.
